# EUROPÄISCHE PATENTSCHRIFT

(11) **EP 3 626 173 B1**
(45) Veröffentlichungstag und Bekanntmachung des Hinweises auf die Patenterteilung: **05.05.2021**
(21) Anmeldenummer: 18195337.3
(22) Anmeldetag: 19.09.2018
(51) Int. Cl.: A61B 6/00, A61B 6/04

(54) **VERFAHREN ZUR BEWEGUNGSKORREKTUR EINES REKONSTRUIERTEN DREI-DIMENSIONALEN BILDDATENSATZES, BIPLAN-RÖNTGENEINRICHTUNG, COMPUTERPROGRAMM UND ELEKTRONISCH LESBARER DATENTRÄGER**
METHOD FOR MOTION CORRECTION OF A RECONSTRUCTED THREE-DIMENSIONAL IMAGE DATASET, BIPLAN X-RAY DEVICE, COMPUTER PROGRAM AND ELECTRONICALLY READABLE DATA CARRIER
PROCÉDÉ DE CORRECTION DE DÉPLACEMENT D'UN ENSEMBLE DE DONNÉES D'IMAGE TRIDIMENSIONNEL RECONSTRUIT, DISPOSITIF À RAYONS X BIPLAN, PROGRAMME INFORMATIQUE ET SUPPORT DE DONNÉES LISIBLE ÉLECTRONIQUEMENT

(43) Veröffentlichungstag der Anmeldung: 25.03.2020
(73) Patentinhaber: Siemens Healthcare GmbH, 91052 Erlangen (DE)
(72) Erfinder: Regensburger, Alois, 91058 Erlangen (DE)

(56) Entgegenhaltungen:
- EP-A2- 1 523 951
- DE-B3-102012 202 648
- BEYAR R ET AL: "Prospective Motion Correction of X-Ray Images for Coronary Interventions", IEEE TRANSACTIONS ON MEDICAL IMAGING, IEEE SERVICE CENTER, PISCATAWAY, NJ, US, Bd. 24, Nr. 4, 2. April 2005 (2005-04-02), Seiten 441-450, XP011129442, ISSN: 0278-0062, DOI: 10.1109/TMI.2004.839679

## Beschreibung

Verfahren zur Bewegungskorrektur eines rekonstruierten dreidimensionalen Bilddatensatzes, Biplan-Röntgeneinrichtung, Computerprogramm und elektronisch lesbarer Datenträger Die Erfindung betrifft ein Verfahren zur Bewegungskorrektur eines aus einer Mehrzahl von zweidimensionalen Projektionsbildern, die mit einer Röntgeneinrichtung aufgenommen werden, rekonstruierten dreidimensionalen Bilddatensatzes hinsichtlich einer Bewegung in einem durch den Bilddatensatz abgedeckten Untersuchungsbereich eines Patienten. Daneben betrifft die Erfindung eine Biplan-Röntgeneinrichtung, ein Computerprogramm und einen elektronisch lesbaren Datenträger.

Aus zweidimensionalen, in unterschiedlichen Aufnahmegeometrien, insbesondere unter unterschiedlichen Projektionswinkeln, aufgenommenen zweidimensionalen Röntgen-Projektionsbildern einer Röntgeneinrichtung können, wie grundsätzlich bekannt ist, beispielsweise durch gefilterte Rückprojektion und/oder algebraische Rekonstruktion, dreidimensionale Bilddatensätze des in den Projektionsbildern zu sehenden Untersuchungsbereichs rekonstruiert werden. Während es dabei zum einen bekannt ist, Computertomographie-Röntgeneinrichtungen einzusetzen, bei denen der Durchlauf eines abzudeckenden Projektionswinkelintervalls durch die Aufnahmeanordnung, umfassend einen Röntgenstrahler und einen Röntgendetektor, äußerst schnell möglich ist, werden dreidimensionale Bilddatensätze immer häufiger auch an Arbeitsplätzen erzeugt, an denen beispielsweise eine Röntgeneinrichtung mit einem C-Bogen bereit steht, insbesondere im Rahmen von minimalinvasiven Interventionen an dem Patienten, wo beispielsweise der Fortschritt einer Untersuchung und/oder Behandlung überwacht werden soll. Die Aufnahme von Projektionsbildern mit einer C-Bogen-Röntgeneinrichtung und die Rekonstruktion eines dreidimensionalen Bilddatensatzes hieraus ist bei der Anmelderin auch unter dem Namen "DynaCT" bekannt.

Ein grundsätzliches Problem bei der zeitlich versetzten Aufnahme von zweidimensionalen Projektionsbildern mit einer Röntgeneinrichtung ist, dass Bewegungen des Patienten, beispielsweise periodische Bewegungen wie die Atembewegung und/oder der Herzschlag, aber auch sonstige Patientenbewegungen, zu einer Verschiebung aufzunehmender anatomischer Strukturen im Untersuchungsbereich führen, was zu Artefakten/undeutlicher Wiedergabe anatomischer Strukturen ("Verschwimmen") führen kann und im Extremfall den dreidimensionalen Bilddatensatz für eine diagnostische oder sonstige Auswertung unbrauchbar machen kann. Daher wurden im Stand der Technik bereits verschiedene Ansätze vorgeschlagen, um Patientenbewegungen während der Aufnahme der Projektionsbilder zu minimieren und/oder Bewegungsartefakte in dem dreidimensionalen Bilddatensatz zu verringern oder gar gänzlich zu vermeiden (Bewegungskorrektur).

Ein erster Ansatz betrifft die Vermeidung von Atembewegungen. Hierzu kann beispielsweise vorgesehen sein, eine Beatmung von Patienten unter Vollnarkose zeitweise abzuschalten, andererseits kann vorgesehen sein, dass bei kooperativen Patienten ohne Narkose ein aktives Atemanhalten für den Zeitraum der Aufnahme der Projektionsbilder gefordert wird. Beide Vorgehensweisen sind nicht immer sinnvoll einsetzbar und betreffen nur die Atembewegung.

In einem Artikel von C. Syben et al., "Joint calibration and motion estimation in weight-bearing conebeam CT of the knee joint using fiducial markers," 2017 IEEE 14th International Symposium on Biomedical Imaging (ISBI 2017), Melbourne, VIC, 2017, pp. 494-497. doi: 10.1109/ISBI.2017.7950568, wird vorgeschlagen, Bezugsmarker bei Bewegungen eines Knies während der Aufnahme der Projektionsbilder nachzuverfolgen.

Ein weiterer Artikel von A. Sindel et al., "Respiratory Motion Compensation for C-Arm CT Liver Imaging", in: Handels H., Deserno T., Meinzer HP., Tolxdorff T. (eds) "Bildverarbeitung für die Medizin 2015. Informatik aktuell." Springer Vieweg, Berlin, Heidelberg, schlägt vor, sichtbare Strukturen in den Projektionsbildern über deren Aufnahmezeitraum nachzuverfolgen.

Schließlich wurde auch in einem Artikel von S. Rit et al., "On-the-fly motion-compensated cone-beam CT using an a priori model of the respiratory motion", Med. Phys., 36: 2283-2296. doi:10.1118/1.3115691 vorgeschlagen, die Bewegungen eines Atemzyklus zuvor aufzunehmen und zu modellieren, um dann eine entsprechende Kompensation vorzunehmen, was aber eine sehr stabile und regelmäßige Atmung voraussetzt, was gerade bei Patienten mit schwacher Sedierung oftmals nicht gegeben ist.

Problematisch bei den zuvor genannten Ansätzen der Verfolgung von sichtbaren Strukturen bzw. Bezugsmarkern ist, dass nur zweidimensionale Positionsinformationen erhalten werden, die zu aufeinanderfolgenden Zeitpunkten auch bezüglich anderer Aufnahmegeometrien, insbesondere anderer Projektionswinkel, vorliegen, und daher nicht unmittelbar fehlerfrei in Beziehung gesetzt werden können.

DE 10 2013 202 313 A1 betrifft ein Verfahren und eine Vorrichtung zur Korrektur von Bewegungsartefakten bei einem computertomographischen Bild. Darin wird vorgeschlagen, zunächst ein vorläufiges dreidimensionales computertomographisches Bild zu rekonstruieren, um eine mittlere Position eines Untersuchungsbereichs zu bestimmen. Danach wird mit Hilfe wenigstens eines Bildvolumenblocks, der aus vorgebbaren Projektionsbildern gebildet ist, mittels eines Optimierungsverfahrens die Bewegung des Untersuchungsbereichs des Untersuchungsobjekts in dem wenigstens einen Bildvolumenblock geschätzt. Ein ähnlicher, ebenso bildbasierter Ansatz, der anderweitig vorgeschlagen wurde, betrifft ebenso eine initiale dreidimensionale Rekonstruktion eines hochkontrastierten Objekts, beispielsweise eines Gefäßbaums der Leber mit arteriellem Kontrast, wonach iterativ durch Registrierung der einzelnen zweidimensionalen Projektionsbilder zum dreidimensionalen Objekt die jeweilige Bewegung in dem Projektionsbild geschätzt werden soll. Hieraus kann ein zeitliches Bewegungsfeld errechnet werden, welches anschließend in dem Projektionsbildern kompensiert wird, wonach eine erneute, bewegungsunterdrückte dreidimensionale Rekonstruktion mit erhöhter Qualität erfolgt.

Auch bei diesen bildbasierten Ansätzen besteht das wesentliche Problem, dass zu einem Zeitpunkt in den jeweiligen Projektionsbildern nur zweidimensionale Informationen vorliegen und zudem von einem vorläufig erzeugten dreidimensionalen Rekonstruktionsdatensatz ausgegangen wird, der nicht bewegungskorrigiert ist und daher auch bereits Fehler aufweisen kann.

US 2017/0273665 A1 betrifft die Posenbestimmung eines Ultraschall-Transducers. Inertial-Messeinheiten werden auf dem Ultraschall-Transducer positioniert, die mit anderen Positionssensoren, beispielsweise Röntgen, kombiniert werden sollen, um die Genauigkeit und/oder die Rate, mit der Poseninformationen vorliegen, zu verbessern. Ferner wurden im Zusammenhang der Ultraschallbildgebung bereits Bezugsmarker vorgeschlagen, mit welchen aus einer einzigen Fluoroskopieaufnahme eine Orientierung eines TEE-Sensors bestimmt werden kann, vergleiche hierzu den Artikel von Tobias Heimann et al., "Real-time ultrasound transducer localization in fluoroscopy images by transfer learning from synthetic training data", Medical image analysis, Vol. 18, Issue 8, Seiten 1320 bis 1328.

Ferner offenbart DE 10 2012 202 648 B3 ein Verfahren zur Abtastung kleiner beweglicher Objekte mittels eines Biplan-Angiographiesystems, wobei eine bewegungskorrigierte dreidimensionale Rekonstruktion ermöglicht wird. Im Artikel von G. Shechter et al., "Prospective motion correction of X-ray images for coronary interventions," in IEEE Transactions on Medical Imaging, Vol. 24, Nr. 4, S. 441-450, April 2005, wird eine Methode zur prospektiven Bewegungskorrektur bei Röntgenbildgebung am Herzen offenbart.

Der Erfindung liegt die Aufgabe zugrunde, eine verbesserte Bewegungskorrektur für aus über einen Zeitraum aufgenommenen zweidimensionalen Projektionsbildern zu rekonstruierende Bilddatensätze anzugeben.

Zur Lösung dieser Aufgabe sind bei einem Verfahren der eingangs genannten Art erfindungsgemäß die Schritte gemäss des Anspruchs 1 vorgesehen.

Eine grundlegende Idee der vorliegenden Erfindung ist es also, eine Biplan-Röntgeneinrichtung zu verwenden, die die zumindest im Wesentlichen gleichzeitige Aufnahme von Projektionsbildern mit unterschiedlichen Aufnahmegeometrien, konkret also unterschiedlichen Projektionswinkeln, erlaubt. Bei der Biplan-Röntgeneinrichtung handelt es sich bevorzugt um eine Biplan-Röntgeneinrichtung mit zwei C-Bögen, an denen jeweils sich gegenüberliegend ein Röntgendetektor und ein Röntgenstrahler angeordnet sind, die jeweils eine Aufnahmeanordnung bilden. Die beiden Ebenen der Biplan-Röntgeneinrichtung sind bevorzugt um 90° gegeneinander versetzt; denkbar ist es jedoch auch, dies einstellbar zu gestalten, jedoch hat sich gezeigt, dass eine Versetzung von 90° zu optimalen Ergebnissen bei der dreidimensionalen Positionsbestimmung des Markerobjekts führt. Mittels der Biplan-Röntgeneinrichtung, insbesondere der Biplan-C-Bogen-Röntgeneinrichtung, nehmen mithin beide Röntgenebenen, also beide Aufnahmeanordnungen bestehend aus einem Röntgendetektor und einem Röntgenstrahler, simultan Projektionsbilder aus jeweils einem Teilwinkelbereich auf. Das bedeutet, die beiden Aufnahmeanordnungen führen gleichzeitig eine Rotationsbewegung über jeweils einen Teil des für die Rekonstruktion benötigten Gesamtwinkelbereichs durch. Der dreidimensionale Bilddatensatz kann dann auf Basis der Projektionsbilder beider Aufnahmeanordnungen, mithin beider Röntgenebenen, rekonstruiert werden.

Bei dem Markerobjekt handelt es sich bevorzugt um ein Hochkontrastobjekt, das sich deutlich in den Bilddaten der zweidimensionalen Projektionsbilder abhebt. Das Markerobjekt befindet sich während der Aufnahme der Projektionsbilder im aufzunehmenden Untersuchungsbereich. Wichtig ist, dass das Markerobjekt als Hochkontrastobjekt der Bildverarbeitung möglichst unkompliziert in den zweidimensionalen Projektionsbildern lokalisiert werden kann. Zur Lokalisierung des Markerobjekts können im Stand der Technik grundsätzlich bekannte Bildverarbeitungstechniken, insbesondere Segmentierungstechniken, eingesetzt werden, welche insbesondere bei Hochkontrastobjekten problemlos vollständig automatisiert diese lokalisieren können.

Die der Erfindung zu Grunde liegende Idee ist es nun, dass auf Grund der gleichzeitigen Aufnahme von Projektionsbildern unter Verwendung unterschiedlicher Aufnahmegeometrien Informationen über das Markerobjekt aus unterschiedlichen Richtungen, definiert durch den Projektionswinkel, vorliegen. Mit anderen Worten werden die n-ten Projektionsbilder P_{A} (tₙ), P_{B} (tₙ) von den Aufnahmeanordnungen A und B gleichzeitig (oder äußerst schnell hintereinander folgend) zum Zeitpunkt tₙ aufgenommen. Nachdem über eine geeignete Bildverarbeitungstechnik, insbesondere Bilderkennung, das Markerobjekt, welches sowohl in P_{A} (tₙ)als auch in P_{B} (tₙ) sichtbar ist, lokalisiert wurde, kann die dreidimensionale Position des Markerobjekts zum Zeitpunkt (tₙ) über die entsprechenden geometrischen Zusammenhänge (wie Epipolargeometrie) ebenso automatisch berechnet werden. Dabei wird mithin ausgenutzt, dass zumindest für einen bestimmten, identifizierbaren Punkt die Informationen aus zwei, insbesondere senkrecht aufeinander stehenden, Richtungen ausreichend sind, um die dreidimensionale Position zu ermitteln, sozusagen also zu triangulieren.

Damit liegt dann dreidimensionale Positionsinformationen bezüglich des Markerobjekts über alle Zeitpunkte (tₙ) vor, die einen Bewegungsverlauf beschreiben und, gegebenenfalls nachbearbeitet, mithin eine Bewegungsinformation bilden, aus der notwendige Korrekturen, beispielsweise in den einzelnen Projektionsbildern, abgeleitet werden können.

Dabei ist insbesondere vorteilhaft, dass verschiedenste Arten von Bewegungen auf diese Art und Weise genau abgegriffen und durch die Bewegungsinformation äußerst genau dreidimensional beschrieben werden können. Mit anderen Worten ist die vorliegende Erfindung nicht festgelegt auf periodische Bewegungen eines Patienten, beispielsweise die Atembewegung, sondern erfasst letztlich jede stattfindende Bewegung im Untersuchungsbereich, die das Markerobjekt betrifft. Die dreidimensionalen Positionsinformationen, die dieser Bewegungsinformation zu Grunde liegen, können hochgenau ermittelt werden, nachdem gleichzeitige Informationen aus unterschiedlichen Projektionswinkeln vorliegen und vorteilhaft zusammengefasst werden. Auf diese Weise lässt sich eine hochqualitative Kompensation von Bewegungseffekten auf den dreidimensionalen Bilddatensatz durchführen, indem letztlich die Bewegungskorrektur so angewandt wird, dass sich sämtliche Projektionsbilder oder zumindest alle Bilddaten des dreidimensionalen Bilddatensatzes auf denselben Bewegungszustand im Untersuchungsbereich beziehen. Es werden bessere dreidimensionale Bilddatensätze bei Aufnahmen von beweglichen Organen erhalten, ohne dass eine Atemkontrolle oder sonstige Maßnahmen nötig sind.

Dabei sei an dieser Stelle noch angemerkt, dass bei Biplan-Röntgeneinrichtungen, beispielsweise Biplan-Röntgeneinrichtungen mit zwei C-Bögen, selbstverständlich eine Registrierung der Aufnahmeanordnungen zueinander vorliegt, nachdem ein gemeinsames Koordinatensystem der Röntgeneinrichtung besteht und viele Anwendungen auch anderer Art diese Registrierung ohnehin benötigen. Auch die Winkelstellungen und dergleichen der C-Bögen bzw. der Aufnahmeanordnungen im Allgemeinen sind in einer Steuereinrichtung der Röntgeneinrichtung selbstverständlich bekannt. Auf diese Weise liegen alle Informationen vor, um Bildinformationen aus gleichzeitig aufgenommenen Projektionsbildern zueinander in Beziehung zu setzen und mithin die dreidimensionale Positionsinformation aus den zweidimensionalen Ortsinformationen zu gewinnen.

Die Bewegungskorrektur wird bevorzugt vor der Rekonstruktion des dreidimensionalen Bilddatensatzes auf die einzelnen Projektionsbilder angewendet, wobei, wie bereits beschrieben, bevorzugt ein bestimmter Bewegungszustand im Untersuchungsbereich ausgewählt wird, bezüglich dessen die Bewegungsinformation die jeweiligen Abweichungen beschreibt, welche zur Korrektur einzelner Projektionsbilder zu bestimmten Aufnahmezeitpunkten eingesetzt werden können. Grundsätzlich sind derartige Möglichkeiten zur Bewegungskorrektur im Stand der Technik bereits bekannt und müssen daher hier nicht mehr ausgeführt werden. Ferner sind bereits Bewegungskorrekturansätze bekannt geworden, die bei der Rekonstruktion des dreidimensionalen Bilddatensatzes ansetzen und selbstverständlich ebenso im Rahmen der vorliegenden Erfindung angewandt werden können.

Als das wenigstens eine Markerobjekt kann ein anatomisches Markerobjekt und/oder ein in oder an dem Patienten zusätzlich vorgesehener Bezugsmarker, insbesondere ein Bezugsmarker eines in den Patienten eingeführten medizinischen Instruments, verwendet werden. Beispiele für Hochkontrastobjekte, die als Markerobjekte verwendet werden können, sind beispielsweise röntgensichtbare Bezugsmarker eines Katheters, röntgensichtbare Marker eines Führungsdrahtes, Endoskopköpfe, implantierte Metallobjekte, Kontrastmittelansammlungen im Körper, leicht erkennbare Knochenstrukturen und dergleichen.

Des Weiteren ist gemäß der vorliegenden Erfindung vorgesehen, dass für ein Markerobjekt auf Grund dessen Formgebung wenigstens eine Orientierung des Markerobjekts als Teil der Positionsinformationen bestimmt und bei dem Bewegungsverlauf berücksichtigt wird. Darüber hinaus können bei mehreren Markerobjekten deren relative Positionsinformationen zur Ableitung einer Orientierungsinformation wenigstens eines Teils des Untersuchungsbereichs verwendet werden. In beiden Fällen wird es mithin ermöglicht, auch Rotationen als Bewegungen in der Bewegungskorrektur zu berücksichtigen. Konkret ist es also zum einen denkbar, eine Drehbewegung mit in der Bewegungsinformation abzubilden, wenn die zweidimensionalen Projektionsbilder des Markerobjekts auch Informationen über dessen räumliche Orientierung zu den jeweiligen Aufnahmezeitpunkten liefern können. Diesbezüglich wurden bereits Formgebungen für Bezugsmarker vorgeschlagen, die eine derartige Bestimmung der Orientierung ermöglichen sollen, gegebenenfalls sogar bereits aus einem einzigen Projektionsbild. Dann ist sogar ein Abgleich bestimmter Orientierungen aus dem Paar von Projektionsbildern gegeneinander möglich. Auf diese Weise liegen dann Orientierungsinformationen als Teil der Positionsinformationen vor, so dass der Bewegungsverlauf nicht nur eine Positionsveränderung, sondern auch eine Orientierungsveränderung über den Aufnahmezeitraum der Projektionsbilder beschreibt. Mit anderen Worten können die einzelnen Positionsinformationen dann als die Pose des Markerobjekts beschreibend ermittelt werden. In gewisser Weise können Drehbewegungen von Teilen des Untersuchungsbereichs gegeneinander auch festgestellt werden, wenn eine Mehrzahl von Markerobjekten betrachtet wird, nachdem bei nicht fester Position der Markerobjekte zueinander Positionsveränderungen auftreten können, die auf einen kompressiven und/oder aber rotatorischen Anteil der Bewegungen schließen lassen, der entsprechend beschrieben werden kann.

Es sei in diesem Zusammenhang, insbesondere bezüglich der Bestimmung einer Orientierungsinformation für einzelne Markerobjekte, noch darauf hingewiesen, dass künstliche Bezugsmarker, die also nicht anatomischen Strukturen entsprechen, bevorzugt während der Aufnahmezeit mit der Anatomie des Untersuchungsbereichs bewegungsgekoppelt sein sollten und idealerweise auch keine Eigenbewegung, die unabhängig von der Anatomie erfolgt, aufweisen sollten. Mithin sollte beispielsweise der Vorschub eines Katheters mit Bezugsmarken während der Aufnahme der Projektionsbilder kurzzeitig unterlassen werden und dergleichen.

Zweckmäßigerweise kann der Bewegungsverlauf vor der Durchführung der Bewegungskorrektur geglättet werden. Auf diese Weise können insbesondere Messfehler oder dergleichen bzw. deren Einfluss reduziert werden. Alternativ oder zusätzlich ist es selbstverständlich auch denkbar, eine gezielte Ausreißerdetektion vorzunehmen und diese Ausreißer der Betrachtung zu entziehen. Werden Ortsinformationen bzw. Positionsinformationen als Ausreißer detektiert und entfernt, können Ersatz-Positionsinformationen für diesen Aufnahmezeitpunkt beispielsweise durch Interpolation des Bewegungsverlaufs ermittelt werden. Ohnehin kann es insgesamt zweckmäßig sein, für den gesamten Aufnahmezeitraum durch Interpolation den Bewegungsverlauf als Teil der Bewegungsinformation vollständig zu ermitteln.

Es kann in einer einfachen Ausgestaltung der vorliegenden Erfindung vorgesehen sein, dass bei der Bewegungskorrektur die Bewegung des Markerobjekts als die Bewegung des Untersuchungsbereichs angenommen wird. Eine derartige Ausgestaltung ist insbesondere dann zweckmäßig, wenn es sich bei dem Untersuchungsbereich um einen sich insgesamt im Wesentlichen gleichförmig bewegenden Untersuchungsbereich handelt. Werden mehrere Markerobjekte betrachtet und soll deren Bewegung als die Bewegung des Untersuchungsbereichs aufgefasst werden, kann auch eine statistische Verarbeitung der Bewegungsverläufe der verschiedenen Markerobjekte vorgenommen werden, beispielsweise durch Bildung von Mittelwerten. Die Annahme, dass sich der Untersuchungsbereich (oder zumindest die wesentlichen anatomischen Strukturen im Untersuchungsbereich) gleichmäßig mit den Markerobjekten bewegen, kann beispielsweise bei Untersuchungen im Bereich von Knochen zweckmäßig sein, aber auch bei Untersuchungen im Bauchraum, wo beispielsweise die Atembewegung einen relativ gleichmäßigen Einfluss hat, kann eine entsprechende Näherung zulässig sein.

Es ist im Rahmen der vorliegenden Erfindung jedoch auch denkbar, die Bewegungsinformationen zur Anpassung eines Bewegungsmodells des Untersuchungsbereichs zu verwenden, wobei die vorzunehmenden lokalen Einzelbewegungskorrekturen aus dem Bewegungsmodell ermittelt werden. Beispielsweise kann als Bewegungsmodell ein Biomechanikmodell verwendet werden. Eine derartige Ausgestaltung bietet sich insbesondere dann an, wenn mehrere Markerobjekte und deren Bewegungsverlauf detektiert werden. Dann kann die Bewegungsinformation genutzt werden, um ein Bewegungsmodell, insbesondere ein Biomechanikmodell, zu treiben, so dass das Bewegungsfeld interessierender anatomischer Strukturen und/oder des Untersuchungsbereichs allgemein ermittelt werden kann. Insbesondere erlaubt die Verwendung eines Bewegungsmodells auch die Interpolation von Bewegungen an vom Markerobjekt bzw. den Markerobjekten entfernten Punkten im Untersuchungsbereich. Die Verwendung eines Bewegungsmodells ist insbesondere dann zweckmäßig, wenn besonders ungleichförmig bewegte Körperbereiche/Untersuchungsbereiche betrachtet werden.

Besonders vorteilhaft kann die vorliegende Erfindung angewendet werden, wenn die Aufnahme der Projektionsbilder zur Überwachung und/oder Vorbereitung und/oder Nachbereitung eines minimalinvasiven Eingriffs an dem Patienten erfolgt. Dabei haben sich besondere Vorteile bei der Durchführung einer endoskopisch retrograden Cholangiopankreatikographie (ERCP) erwiesen. Gerade bei minimalinvasiven Eingriffen sind hochqualitative dreidimensionale Bilddaten äußerst nützlich, beispielsweise auch was die Beobachtung eines Behandlungsfortschritts und/oder die korrekte Positionierung von Implantaten und/oder sonstigen medizinischen Instrumenten angeht.

Neben dem Verfahren betrifft die vorliegende Erfindung auch eine Biplan-Röntgeneinrichtung, aufweisend zwei insbesondere jeweils an einem C-Bogen angeordnete Aufnahmeanordnungen, die jeweils einen Röntgenstrahler und einen Röntgendetektor umfassen, und eine zur Durchführung des erfindungsgemäßen Verfahrens ausgebildete Steuereinrichtung. Die Steuereinrichtung weist dabei insbesondere wenigstens einen Prozessor und wenigstens ein Speichermittel auf. Beispielsweise kann die Steuereinrichtung zur Durchführung des erfindungsgemäßen Verfahrens, neben einer üblichen Aufnahmeeinheit zur Steuerung der Aufnahme von zweidimensionalen Projektionsbildern entlang vorgegebener, die Winkelbereiche abdeckender Aufnahmetrajektorien, eine Lokalisierungseinheit zur Lokalisierung des wenigstens einen Markerobjekts, eine Bestimmungseinheit zur Ermittlung der Positionsinformationen, eine Ermittlungseinheit zur Ermittlung der Bewegungsinformation und eine Korrektureinheit zur Bewegungskorrektur des Bilddatensatzes, insbesondere der einzelnen Projektionsbilder vor der Rekonstruktion, aufweisen. Weitere Funktionseinheiten können, beispielsweise hinsichtlich verschiedener Ausgestaltungen, zusätzlich vorgesehen sein.

Sämtliche Ausführungen bezüglich des erfindungsgemäßen Verfahrens gelten analog für die erfindungsgemäße Biplan-Röntgeneinrichtung. Bei dieser kann es sich im Übrigen bevorzugt um eine Biplan-Röntgeneinrichtung mit zwei C-Bögen handeln, wobei an jeweils einem der C-Bögen eine der Aufnahmeanordnungen angeordnet ist.

Ein erfindungsgemäßes Computerprogramm ist beispielsweise direkt in einen Speicher einer Steuereinrichtung einer Biplan-Röntgeneinrichtung ladbar und weist Programmmittel auf, um die Schritte eines erfindungsgemäßen Verfahrens auszuführen, wenn das Computerprogramm in der Steuereinrichtung der Röntgeneinrichtung ausgeführt wird. Das Computerprogramm kann auf einem erfindungsgemäßen elektronisch lesbaren Datenträger gespeichert sein, welcher mithin darauf gespeicherte elektronisch lesbare Steuerinformationen umfasst, welche zumindest ein genanntes Computerprogramm umfassen und derart ausgestaltet sind, dass sie bei Verwendung des Datenträgers in einer Steuereinrichtung einer Biplan-Röntgeneinrichtung ein erfindungsgemäßes Verfahren durchführen. Bei dem Datenträger kann es sich insbesondere um einen nichttransienten Datenträger, beispielsweise eine CD-ROM, handeln.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: einen Ablaufplan eines Ausführungsbeispiels des erfindungsgemäßen Verfahrens,
- Fig. 2: eine Prinzipskizze zur Erläuterung der Ermittlung der Positionsinformation, und
- Fig. 3: eine erfindungsgemäße Biplan-Röntgeneinrichtung.

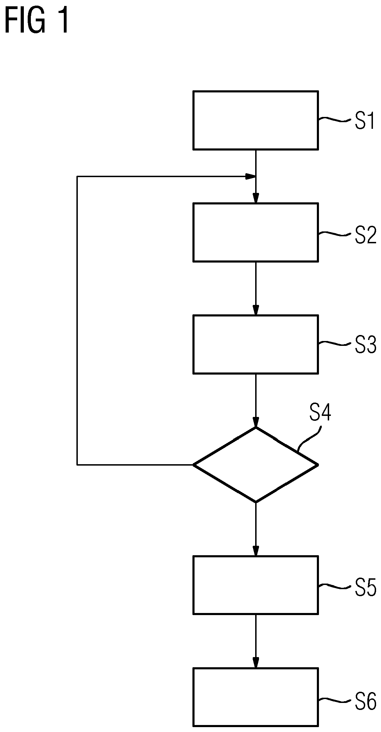

Fig. 1 zeigt einen Ablaufplan eines Ausführungsbeispiels des erfindungsgemäßen Verfahrens. Dabei soll vorliegend eine

Überwachung eines minimalinvasiven Eingriffs, hier konkret einer endoskopisch retrograden Cholangiopankreatikographie, erfolgen, bei der dreidimensionale Bilddatensätze eines Untersuchungsbereichs eines Patienten aus zweidimensionalen Projektionsbildern einer hier verwendeten Biplan-Röntgeneinrichtung rekonstruiert werden sollen. Dazu werden in einem Schritt S1 zunächst die Projektionsbilder aufgenommen. Hierzu werden im vorliegenden Ausführungsbeispiel die Aufnahmeanordnungen der Biplan-Röntgeneinrichtung, die jeweils einen Röntgenstrahler und einen Röntgendetektor umfassen, in einem 90°-Winkel zueinander gekoppelt. Sodann überstreichen beide Aufnahmeanordnungen gleichzeitig durch gemeinsame Rotation der C-Bögen, die die Aufnahmeanordnungen tragen, einen jeweiligen Winkelbereich von Projektionswinkeln, der durch die jeweilige Aufnahmeanordnung abgedeckt werden soll. Der sich durch Zusammenfügung der Winkelbereiche ergebende Gesamtwinkelbereich ist ausreichend für eine Rekonstruktion des dreidimensionalen Bilddatensatzes.

Konkret werden im Schritt S1 zu Aufnahmezeitpunkten jeweils gleichzeitig zweidimensionale Projektionsbilder mit beiden Aufnahmeanordnungen aufgenommen, so dass Paare von Projektionsbildern entstehen, die zum selben Aufnahmezeitpunkt aufgenommen wurden, jedoch in Aufnahmegeometrien, deren Projektionswinkel um 90° versetzt sind.

Bevor allerdings die Rekonstruktion des dreidimensionalen Bilddatensatzes aus den zweidimensionalen Projektionsbildern erfolgt, soll eine Bewegungskorrektur, vorliegend unmittelbar in den zweidimensionalen Projektionsbildern, erfolgen. Hierzu wird ein Markerobjekt innerhalb des Untersuchungsbereichs des Patienten genutzt. Das Markerobjekt zeichnet sich durch hohen Kontrast gegenüber den umliegenden anatomischen Strukturen aus und kann selbst eine anatomische Struktur, beispielsweise ein deutlich erkennbarer Knochen, sein, aber auch ein medizinisches Instrument, beispielsweise ein Implantat, oder ein daran angeordneter, röntgenintransparenter Bezugsmarker, beispielsweise ein Bezugsmarker an einem Endoskop, das zur endoskopisch retrograden Cholangiopankreatikographie genutzt wird. Hierbei kann im Übrigen auch der Endoskopkopf selber als Markerobjekt dienen. Nachdem das Markerobjekt für jedes Paar von Projektionsbildern, das zu einem Aufnahmezeitpunkt aufgenommen wurde, aus unterschiedlichen Richtungen abgebildet wird, lassen sich durch Verwendung der Epipolargeometrie dreidimensionale Ortsinformationen des Markerobjekts für diesen Aufnahmezeitpunkt ermitteln.

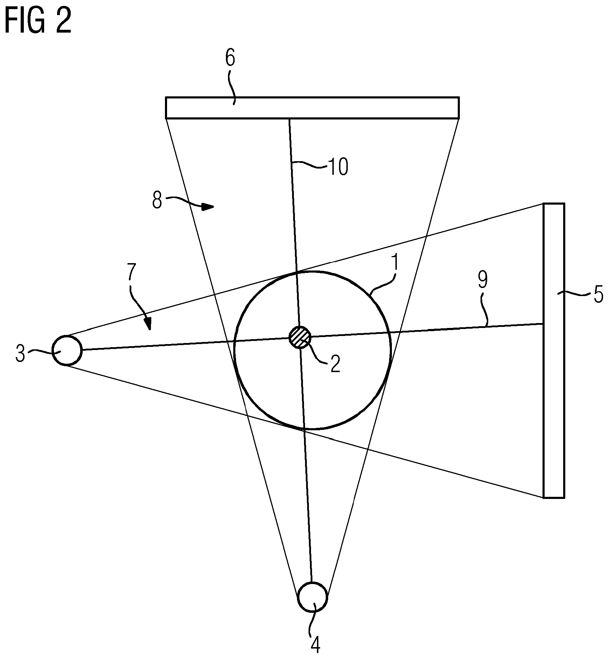

Dies ist schematisch in Fig. 2 angedeutet. Gezeigt ist zum einen der Untersuchungsbereich 1 des Patienten, innerhalb dessen sich das Markerobjekt 2 befindet. Gezeigt sind ferner die beiden Aufnahmeanordnungen mit ihren jeweiligen Röntgenstrahlern 3, 4 und ihren jeweiligen Röntgendetektoren 5, 6. Auch die jeweiligen Strahlenfelder 7, 8 sind gezeigt. Wie aus den Projektionslinien (entspricht Strahlverläufen) 9 und 10 ersichtlich ist, wird das Markerobjekt 2 in den jeweiligen Projektionsbildern der Röntgendetektoren 5, 6 an bestimmten Positionen abgebildet.

Sind diese zweidimensionalen Positionen in den jeweiligen Projektionsbildern eines Paars eines Aufnahmezeitpunkts bekannt, so können aus den ferner der Steuereinrichtung der Röntgeneinrichtung bekannten Aufnahmegeometrien entsprechende dreidimensionale Positionsinformationen bestimmt werden.

Dies passiert im Ablaufplan gemäß Fig. 1 in den Schritten S2 und S3. Im Schritt S2 werden Bildverarbeitungstechniken eingesetzt, insbesondere Segmentierungstechniken, um das ein Hochkontrastobjekt bildende Markerobjekt 2 in den Projektionsbildern eines Paares, das einem Aufnahmezeitpunkt zugeordnet ist, zu lokalisieren, um somit eine zweidimensionale Ortsinformation des Markerobjekts 2 für beide Projektionsbilder zu erhalten. Optional können, beispielsweise falls die Formgebung des Markerobjekts 2 dies erlaubt, auch aus den zweidimensionalen Ortsinformationen beider Projektionsbilder gemeinsam, gegebenenfalls jedoch auch aus den Lokalisierungsdaten jedes einzelnen Projektionsbildes, Orientierungen des Markerobjekts 2 für diesen Aufnahmezeitpunkt abgeleitet werden.

In einem Schritt S3 wird durch Epipolargeometrie, wie erläutert, die dreidimensionale Positionsinformation des Markerobjekts 2 ermittelt, letztlich, einfach ausgedrückt, durch Rückprojektion entlang der Strahlen 9, 10 und Auffinden des Schnittpunktes. Wurde auch eine Orientierung ermittelt, kann diese ebenso als Teil der dreidimensionalen Positionsinformation für den Aufnahmezeitpunkt ermittelt werden, so dass die dreidimensionale Positionsformation eine vollständige Pose des Markerobjekts 2 beschreibt.

Selbstverständlich können die Schritte S2 und S3 auch für mehrere Markerobjekte 2 durchgeführt werden, wenn dies gewünscht ist und eine hinreichende Anzahl von derart automatisch detektierbaren Markerobjekten 2 vorliegt. Über die Epipolargeometrie oder andere Methoden aus dem Stand der Technik können hier - falls die Projektionen der mehreren Markerobjekte 2 nicht in den einzelnen Projektionsbildern unterscheidbar sind - die entsprechenden Korrespondenzen zugeordnet werden.

In einem Schritt S4 wird überprüft, ob für weitere Aufnahmezeitpunkte, also weitere Paare von gleichzeitig aufgenommenen Projektionsbildern, dreidimensionale Positionsinformationen bestimmt werden müssen. Ist dies der Fall, werden die Schritte S2, S3 für das nächste Paar von Projektionsbildern wiederholt. Sind jedoch alle Aufnahmezeitpunkte bearbeitet, wird mit einem Schritt S5 fortgefahren.

Dort wird nun aus den dreidimensionalen Positionsinformationen verschiedener Aufnahmezeitpunkte eine einen Bewegungsverlauf des Markerobjekts 2 über den Aufnahmezeitraum, also den Zeitraum, in dem die Aufnahmezeitpunkte liegen, beschreibende Bewegungsinformation ermittelt. Dabei ist vorliegend vorgesehen, den sich aus den einzelnen Aufnahmezeitpunkten ergebenden Bewegungsverlauf zu glätten und/oder eine Ausreißerdetektion durchzuführen, wobei die entsprechenden Ausreißer entfernt werden.

Ist nun anzunehmen, dass sich der Untersuchungsbereich gleichförmig bewegt, kann die (geglättete und von Fehlmessungen bereinigte) Bewegung des Markerobjekts 2 als Bewegung des gesamten Untersuchungsbereichs 1 angenommen werden. Wurden dann mehrere Markerobjekte 2 betrachtet, können deren Bewegungsverläufe statistisch zu einem mittleren Bewegungsverlauf als Bewegungsinformation zusammengefasst werden.

Bei Untersuchungsbereichen 1, in denen sich Teilbereiche gegeneinander bewegen können, kann es zweckmäßig sein, als Bewegungsinformation ein Bewegungsmodell, insbesondere ein Biomechanikmodell, zu parametrieren, wobei dann bevorzugt mehrere Markerobjekte 2 betrachtet werden. Das Bewegungsmodell liefert dann für jeden Punkt innerhalb des Untersuchungsbereichs 1 und für jeden Aufnahmezeitpunkt einen Bewegungszustand zurück.

In einem Schritt S6 kann die Bewegungsinformation dann genutzt werden, um die Bewegungskorrektur durchzuführen, vorliegend für jeden Aufnahmezeitpunkt auf den entsprechenden zweidimensionalen Projektionsbildern. Dabei wird insbesondere ein Referenzbewegungszustand festgelegt, und durch die Bewegungsinformation werden die Abweichungen von diesem Referenzzustand für jeden Aufnahmezeitpunkt festgestellt, so dass die Bilddaten der Projektionsbilder entsprechend korrigiert werden können. Wurden auch Orientierungen mitbestimmt, können diese ebenso einen Teil der Bewegungsinformation bilden und entsprechend berücksichtigt werden.

Nachdem die Korrektur der Projektionsbilder im Schritt S6 abgeschlossen wurde, kann ein dreidimensionaler Bilddatensatz des Untersuchungsbereichs 1 rekonstruiert werden, welcher auf Grund der Vermeidung oder zumindest Reduzierung von Bewegungsartefakten von besonders hoher Qualität ist.

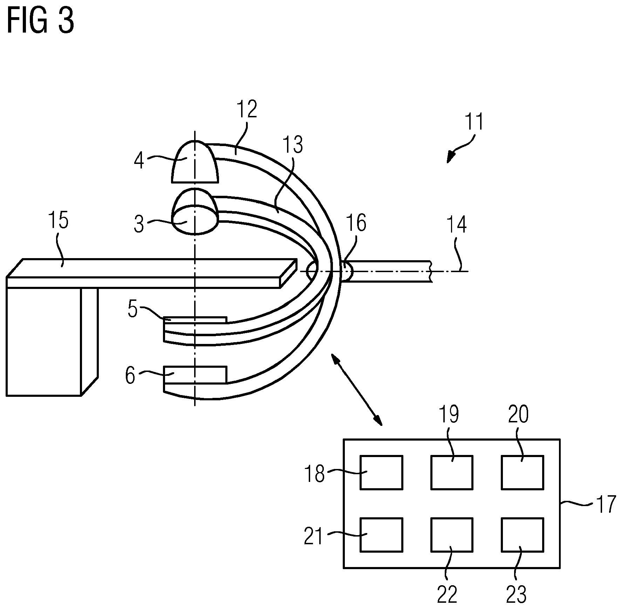

Fig. 3 zeigt schließlich eine Prinzipskizze einer erfindungsgemäßen Biplan-Röntgeneinrichtung 11. Diese weist ersichtlich zwei C-Bögen 12, 13 für die entsprechenden Aufnahmeanordnungen mit den Röntgenstrahlern 3, 4 und den Röntgendetektoren 5, 6 auf. Die C-Bögen 12, 13 sind um eine Rotationsachse 14 gemeinsam oder gekoppelt um einen Patiententisch 15 schwenkbar, wofür eine entsprechende Aktorik 16 vorgesehen sein kann.

Der Betrieb der Biplan-Röntgeneinrichtung 11 wird mittels einer Steuereinrichtung 17 gesteuert, die zur Durchführung des erfindungsgemäßen Verfahrens ausgebildet ist. Hierzu weist die Steuereinrichtung 17 neben einer Aufnahmeeinheit 18 insbesondere auch eine Lokalisierungseinheit 19 zur Durchführung des Schrittes S2, eine Bestimmungseinheit 20 zur Durchführung des Schrittes S3, eine Ermittlungseinheit 21 zur Durchführung des Schrittes S5 und eine Korrektureinheit 22 zur Durchführung des Schrittes S6 auf. Schließlich ist auch eine Rekonstruktionseinheit 23 zur Rekonstruktion des dreidimensionalen Bilddatensatzes aus den entsprechend korrigierten Projektionsbildern vorgesehen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Bewegungskorrektur eines aus einer Mehrzahl von zweidimensionalen Projektionsbildern, die mit einer Röntgeneinrichtung (11) aufgenommen werden, rekonstruierten dreidimensionalen Bilddatensatzes hinsichtlich einer Bewegung in einem durch den Bilddatensatz abgedeckten Untersuchungsbereich (1) eines Patienten, wobei
- eine Biplan-Röntgeneinrichtung (11) mit zwei um einen Winkel, insbesondere 90°, versetzt messenden Aufnahmeanordnungen verwendet wird,
- zur Aufnahme der Projektionsbilder ein jeweiliger Winkelbereich von Projektionswinkeln durch die jeweiligen Aufnahmeanordnungen abgedeckt wird und zumindest im Wesentlichen gleichzeitig Paare von Projektionsbildern des Untersuchungsbereichs (1) zu jeweiligen Aufnahmezeitpunkten aufgenommen werden,
- für jedes Paar von Projektionsbilder wenigstens ein im Untersuchungsbereich (1) liegendes, in den Projektionsbildern abgebildetes Markerobjekt (2) automatisch zur Bestimmung von zweidimensionalen Ortsinformationen lokalisiert wird,
- unter Verwendung der durch die Projektionswinkel beschriebenen Aufnahmegeometrien des jeweiligen Paares der Projektionsbilder eine dreidimensionale Positionsinformation des Markerobjekts (2) bestimmt wird,
- aus den Positionsinformationen verschiedener Aufnahmezeitpunkte eine einen Bewegungsverlauf des Markerobjekts (2) über den Aufnahmezeitraum beschreibende Bewegungsinformation ermittelt wird, und
- die Bewegungsinformation zur Bewegungskorrektur des Bilddatensatzes verwendet wird, **dadurch gekennzeichnet, dass** für das Markerobjekt (2) aufgrund dessen Formgebung wenigstens eine Orientierung des Markerobjekts (2) als Teil der Positionsinformationen bestimmt und bei dem Bewegungsverlauf berücksichtigt wird.

2. Verfahren nach Anspruch 1, **dadurch gekennzeichnet, dass** als das wenigstens eine Markerobjekt (2) ein anatomisches Markerobjet (2) und/oder ein in oder an dem Patienten zusätzlich vorgesehener Bezugsmarker, insbesondere ein Bezugsmarker eines in den Patienten eingeführten medizinischen Instruments, verwendet wird.

3. Verfahren nach einem der vorangehenden Ansprüche, **dadurch gekennzeichnet, dass** der Bewegungsverlauf vor der Durchführung der Bewegungskorrektur geglättet wird.

4. Verfahren nach einem der vorangehenden Ansprüche, **dadurch gekennzeichnet, dass** die Bewegungsinformationen zur Anpassung eines Bewegungsmodells des Untersuchungsbereichs (1) verwendet werden, wobei die vorzunehmenden lokalen Einzelbewegungskorrekturen aus dem Bewegungsmodell ermittelt werden.

5. Verfahren nach Anspruch 4, **dadurch gekennzeichnet, dass** als Bewegungsmodell ein Biomechanikmodell verwendet wird.

6. Verfahren nach einem der vorangehenden Ansprüche, **dadurch gekennzeichnet, dass** die Aufnahme der Projektionsbilder zur Überwachung und/oder Vorbereitung und/oder Nachbereitung eines minimalinvasiven Eingriffs an dem Patienten, insbesondere einer endoskopisch retrograden Cholangiopankreatikographie, erfolgt.

7. Biplan-Röntgeneinrichtung (11), aufweisend zwei insbesondere jeweils an einem C-Bogen angeordnete Aufnahmeanordnungen und eine zur Ausführung eines Verfahrens nach einem der vorangehenden Ansprüche ausgebildete Steuereinrichtung (17) .

8. Computerprogramm, welches die Schritte eines Verfahrens nach einem der Ansprüche 1 bis 6 durchführt, wenn es auf einer Steuereinrichtung (17) einer Röntgeneinrichtung (11) ausgeführt wird.

9. Elektronisch lesbarer Datenträger, auf dem ein Computerprogramm nach Anspruch 8 gespeichert ist.

## Claims

1. Method for motion correction of a three-dimensional image dataset reconstructed from a plurality of two-dimensional projection images acquired by an X-ray device (11), which motion correction relates to a movement in a region under examination (1) of a patient that is covered by the image dataset, wherein
- a biplane X-ray device (11) having two acquisition assemblies that measure at an angular offset, in particular of 90°, is used;
- in order to acquire the projection images, each of the acquisition assemblies covers an angular range of projection angles, and pairs of projection images of the region under examination (1) are acquired at least substantially simultaneously at each acquisition time instant;
- for each pair of projection images, at least one marker object (2) lying in the region under examination (1) and imaged in the projection images is automatically localised in order to determine two-dimensional location information;
- three-dimensional position information about the marker object (2) is determined using the acquisition geometries of the particular pair of projection images, which acquisition geometries are defined by the projection angles;
- motion information describing a motion profile of the marker object (2) over the acquisition period is ascertained from the position information at different acquisition time instants; and
- the motion information is used for motion correction of the image dataset, **characterised in that** for the marker object, at least one orientation of the marker object (2) is determined as part of the position information on the basis of its shape, and is included in the motion profile.

2. Method according to claim 1, **characterised in that** an anatomical marker object (2) and/or a reference marker additionally provided in, or on, the patient, in particular a reference marker of a medical instrument inserted into the patient, is used as the at least one marker object (2).

3. Method according to one of the preceding claims, **characterised in that** the motion profile is smoothed before performing the motion correction.

4. Method according to one of the preceding claims, **characterised in that** the motion information is used to adapt a motion model of the region under examination (1), wherein the local individual motion corrections to be performed are ascertained from the motion model.

5. Method according to claim 4, **characterised in that** a biomechanical model is used as the motion model.

6. Method according to one of the preceding claims, **characterised in that** the projection images are acquired for the purpose of monitoring and/or preparing and/or post-processing a minimally invasive intervention on the patient, in particular endoscopic retrograde cholangio-pancreatography.

7. Biplane X-ray device (11) comprising two acquisition assemblies, in particular each arranged on a C-arm, and a control device (17) designed to implement a method according to one of the preceding claims.

8. Computer program, which performs the steps of a method according to one of claims 1 to 6 when it is executed in a control device (17) of an X-ray device (11).

9. Electronically readable data storage medium, on which is stored a computer program according to claim 8.

## Revendications

1. Procédé de correction du déplacement d'un ensemble de données d'images tridimensionnelles reconstruites à partir d'une pluralité d'images de projection bidimensionnelles, enregistrées par un appareil (11) à rayons X, en ce qui concerne un déplacement dans une région (1) à examiner d'un patient recouverte par l'ensemble de données d'image, dans lequel
- on utilise un dispositif (11) à rayons X biplan, ayant deux systèmes d'enregistrement mesurant de manière décalée d'un angle, notamment de 90°,
- pour l'enregistrement des images de projection, on recouvre une région angulaire d'angles de projection par les systèmes d'enregistrement et on enregistre sensiblement en même temps des paires d'images de projection de la région (1) à examiner à des instants d'enregistrement respectifs,
- pour chaque paire d'images de projection, on localise automatiquement, pour la détermination d'informations d'endroit bidimensionnelles, au moins un objet (2) marqueur se trouvant dans la région (1) à examiner et reproduit dans les images de projection,
- en utilisant les géométries d'enregistrement, décrites par les angles de projection, de la paire des images de projection, on détermine une information de position tridimensionnelle de l'objet (2) marqueur,
- à partir des informations de position de divers instants d'enregistrement, on détermine une information de déplacement décrivant une courbe de déplacement de l'objet (2) marqueur pendant le laps de temps d'enregistrement, et
- on utilise l'information de déplacement pour la correction du déplacement de l'ensemble de données d'images, **caractérisé en ce que**, pour l'objet marqueur, on détermine, sur la base de sa conformation, au moins une orientation de l'objet (2) marqueur comme partie des informations de position et on en tient compte dans la courbe de déplacement.

2. Procédé suivant la revendication 1, **caractérisé en ce que** l'on utilise, comme au moins un objet (2) marqueur, un objet (2) marqueur anatomique et/ou un marqueur de référence prévu supplémentairement sur le patient, notamment un marqueur de référence d'un instrument médical introduit dans le patient.

3. Procédé suivant l'une des revendications précédentes, **caractérisé en ce que** l'on lisse la courbe de déplacement avant d'effectuer la correction du déplacement.

4. Procédé suivant l'une des revendications précédentes, **caractérisé en ce que** l'on utilise les informations de déplacement pour adapter un modèle de déplacement de la région (1) à examiner, les corrections locales de déplacement individuel à effectuer étant déterminées à partir du modèle de déplacement.

5. Procédé suivant la revendication 4, **caractérisé en ce que** l'on utilise un modèle de biomécanique comme modèle de déplacement.

6. Procédé suivant l'une des revendications précédentes, **caractérisé en ce que** l'enregistrement des images de projection s'effectue pour le contrôle et/ou le prétraitement et/ou le post-traitement d'une intervention invasive au minimum sur le patient, notamment d'une cholangiopancréatographie rétrograde de façon endoscopique.

7. Dispositif (11) à rayons X biplan, comportant deux systèmes d'enregistrement, disposés notamment chacun sur un arceau en C, et un dispositif (17) de commande constitué pour effectuer un procédé suivant l'une des revendications précédentes.

8. Programme d'ordinateur, qui effectue les stades d'un procédé suivant l'une des revendications 1 à 6, lorsqu'il est réalisé sur un dispositif (17) de commande d'un dispositif (11) à rayons X.

9. Support de données déchiffrables électronique, sur lequel est mis en mémoire un programme d'ordinateur suivant la revendication 8.
